# EUROPEAN PATENT SPECIFICATION

(11) **EP 1 964 514 B1**
(45) Date of publication and mention of the grant of the patent: **13.04.2011**
(21) Application number: 07103263.5
(22) Date of filing: 28.02.2007
(51) Int. Cl.: A61B 5/083, A61M 16/04

(54) **Controlling the operation of a respiratory gas monitor**
Steuerung des Betriebs eines Atemgasüberwachungsgeräts
Contrôle d'exploitation d'un moniteur d'apnée à gaz

(43) Date of publication of application: 03.09.2008
(73) Proprietor: GENERAL ELECTRIC COMPANY, Schenectady, NY 12345 (US)
(72) Inventor: Karlsson, Kai, 00660, Helsinki (FI)
(74) Representative: Laako, Tero Jussi

(56) References cited:
- GB-A- 829 409
- US-A- 4 550 726
- US-A- 4 958 075
- US-A- 5 873 361

## Description

### FIELD OF THE INVENTION

The invention relates to a method for controlling the operation of a respiratory gas monitor, which comprises at least one respiratory gas analyzer, a gas sampling line connected to a breathing tube that feeds respiratory gas to and from a patient, a gas pump for sucking a sample flow of respiratory gas from said breathing tube through said gas sampling line, a power supply and a processing unit, and either a pressure sensor connected to said gas sampling line and generating an electrical signal connected to said processing unit, or at least one other patient monitor device capable to detect values of at least one physiological signal from said patient, said other patient monitor detection-connected to said patient and electrical-signal-connected to said processing unit.

### BACKGROUND OF THE INVENTION

Respiratory Gas Monitors (= RGM) of the diverting type have a gas pump for drawing a flow of sample gas from the airway of the patient to the RGM. The sample gas is conducted from the sampling site to the RGM by a line with typically 2-3 meters length and internal diameter of about 1.2 mm. The mechanical motion needed for the pumping action is generated using an electrical motor or a solenoid mechanism in the pump. Gas pumps also contain valves actuated by pressure differences within the pump. Regardless of the type of the actuator and the valves, moving mechanical parts in the pump are subjected to stresses and wear, which limit the operating life of the pump. The relatively short operating life of the pump - typically in the order of 5000 hours - is often one of the main factors limiting the reliability of a RGM. When the pump in a RGM fails, the sample gas flow may stop completely or decrease to an unacceptable level. In many RGMs, the sample flow is measured and an alarm is given to the user when the flow is too low. The value of such alarm is quite limited, however, because monitoring of respiratory gases is no more possible after a pump failure. In some RGMs, low flow may remain undetected and lead to measurement errors that may compromise patient safety.

In the prior art, the gas pump in a RGM is running always when the RGM is switched on. Alternatively, unnecessary operation of the pump can be prevented by manually setting the RGM to a "Standby" mode, when gas monitoring is not needed. When a patient is again connected to the RGM, the user must manually switch the monitor from the Standby mode to the normal Operation mode. Often the RGM is kept running continuously for long periods of time even if the need for gas monitoring would be only very intermittent. The reason for this is that many RGMs need warm-up time of several minutes, and most users want to avoid waiting for the warm-up by keeping their RGMs continuously on. It is common that the RGM is switched on in the morning or at the beginning of a work shift and turned off in the evening or at the end of the work shift. In some hospitals, the RGMs may even be switched on for 24 hours a day. A substantial part of the operating hours of the gas pump often consists of periods, when there is no need for respiratory gas monitoring. Even if the RGM could be manually switched to Standby mode, users tend to forget it, because they are busy with more important tasks, when disconnecting a patient from the RGM. If a gas pump is running for 12 hours a day, it typically fails about twice in three years, which means that it is one of the main factors limiting the "mean time between failures" of a RGM. The gas pump in a RGM is connected to both the pneumatics in the RGM and to the electronics circuitry. The replacement of a failed pump must be performed very skillfully in order to eliminate risks for new damages to the electronic circuits, such as those caused by electrostatic discharges and new faults in the pneumatics system, such as gas leakages. Thus, only a qualified service technician may change the pump. This means that the replacement is costly and it may take a substantial time to have the RGM repaired after a pump failure.

The patent GB 829,409 discloses a breathing-tube through which the patient is intended to inspire and expire which is connected to an analyser through which gas can be drawn from the tube, or from the patient's respiratory tract. The analyser incorporates a semi-permeable or other restrictive device whereby gas can only enter the analyser from the breathing-tube, or the patient's respiratory tract when a flow is induced by other means such as a suction pump or fan actuated by a source of electric power. Alternatively a pump or fan could be situated in the tube between the analyser and the breathing-tube, or the respiratory tract. Moreover, between the analyser and the pump or fan is connected a valve actuatable by a solenoid. Two spaced points in the breathing-tube are connected through a pair of tubes respectively to opposite sides of a diaphragm of a pressure-sensitive switch, and the arrangement is such that the pressure differential acting on opposite sides of the diaphragm as the patient expires through the tube will cause the switch to close. The switch is in series with an adjustable resistance and the coil of a relay which is connected to a direct-current supply from the power source. Energization of the relay closes the switch and moves the contact piece of the switch to the alternative position. When the switch is closed the condenser is charged at a rate depending on the setting of the resistance to provide a delay before actuation of the relay. When the relay is actuated the switch is changed from the position shown to discharge the condenser quickly through the resistance, and at the same time the switch is closed to energize the solenoid and thereby open the valve. Opening of the valve will cause the suction pump or fan to be placed in operative connection with the analyser so that gas is drawn into the analyser from the breathing-tube, or from the patient's respiratory tract. At the end of expiration the switch opens and the parts return to the positions shown. In use the resistance is so adjusted that gas is only drawn into the analyser during the latter part of the expiration of the patient, normally corresponding to the expiration of the final part of the "end-tidal gas".

The patent US 5,873,361 suggests a method of preventing the formation of dangerous underpressure in a respiratory system, which comprises a respirator connected to the patient, and a sampling device and a gas analyzer separately connected thereto, in which method inspired and expired air is aspirated at a predetermined pressure for sampling through the sampling line. The pressure in the respiratory tube is compared with the ambient pressure, and when an excess underpressure is formed in the gas analyzing unit, the aspiration of the pump is prevented in the respiratory tube.

The patent US 4,550,726 discloses a method and apparatus for detecting an interruption in the supply of breathing gas to a patient by: During a reference time period, sensing the pressure in the patient's breathing gas; Storing, reference breathing information derived from the pressure sensed during the reference time period; After the reference time period, sensing the pressure in the patient's breathing gas; Comparing active breathing information derived from the pressure sensed after the reference time period with the reference breathing information, and; Producing an alarm signal upon detection, during the comparing step, of predetermined variations between the reference breathing information and the active breathing information.

### SUMMARY OF THE INVENTION

The purpose of the present invention is to eliminate unnecessary operation of the sample gas pump in a RGM. Another purpose of the invention is to improve the reliability of the RGM by avoiding unnecessary wear of the gas pump.

According to the first aspect of the invention, when a pressure sensor is pressure-connected to a gas sampling line and electrical-signal-connected to a processing unit: A minimum value and a maximum value in the signal forwarded by said pressure sensor, and representing a minimum pressure and a maximum pressure respectively inside said gas sampling line, are detected by said processing unit; a difference between said minimum value and said maximum value is calculated, and is compared with a predetermined threshold value in/to said processing unit; and if said calculated difference is: below said threshold value said pump is switched off by said processing unit, and over said threshold value said pump is switched on by said processing unit.

According to the second aspect of the invention, when at least one other patient monitor device capable to detect values of at least one physiological signal from said patient, said other patient monitor is detection-connected to said patient and electrical-signal-connected to said processing unit: A minimum value and a maximum value of said one physiological signal are detected by said other patient monitor; a difference between said minimum value and said maximum value is calculated, and is compared with a predetermined threshold value in/to said processing unit; and if said calculated difference is: below said threshold value said pump is switched off by said processing unit, and over said threshold value said pump is switched on by said processing unit.

### BRIEF DESCRIPTION OF THE DRAWINGS

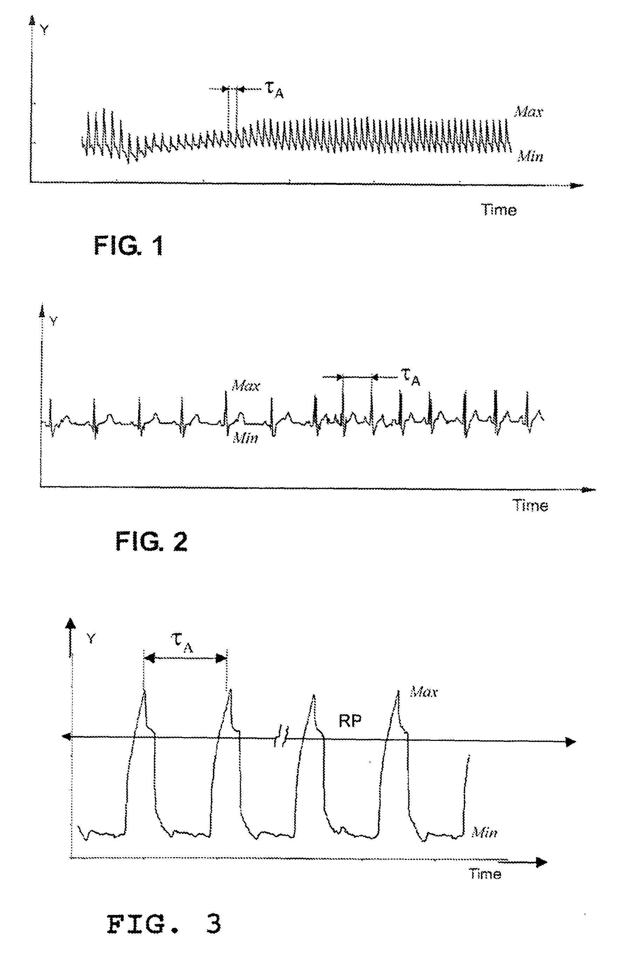

Fig. 1 shows an example of a typical cardiovascular pulse wave signal, i.e. blood pressure or blood flow variations of a patient, which can be attained by e.g. a photoplethysmography. The exact form of the pulses can vary depending on the apparatus used for attaining the signal, but there are always the periodically repeated signal maxima and minima to be utilized by the invention.
Fig. 2 shows an example of a typical electrocardiac or magnetocardiac signal representing cardiac excitation of a patient, which can be attained by an ECG- or a MCG- apparatus. The exact form of the pulses can vary depending on the positioning of the leads for attaining the signal, but there are always the periodically repeated signal maxima and minima to be utilized by the invention.
Fig. 3 shows an example of a typical respiratory signal, i.e. breathing air pressure or breathing airflow variations of a patient, which can be attained by e.g. a photoplethysmographic measuring unit. The exact form of the pulses can vary depending on the apparatus used for attaining the signal, but there are always the periodically repeated signal maxima and minima to be utilized by the invention.

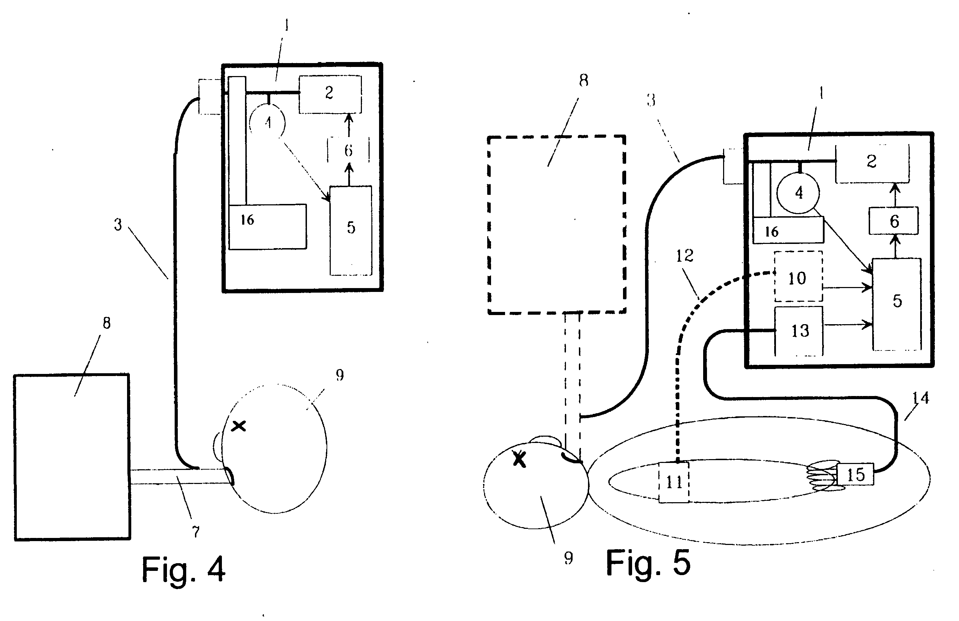

Fig. 4 shows schematically the first embodiment of the invention, in which the pressure sensor inherently present in the respiratory gas monitor device is utilized for detection of the presence/absence of a patient, and hence for switching on/off the gas pump in the respiratory gas monitor device.
Fig. 5 shows schematically the second and the third embodiment of the invention, in which one other patient monitor device - in this case either a pulse oximeter, visualized by continuous lines, or a blood pressure cuff, visualized by broken lines - in most cases present for monitoring patient is utilized for detection of the presence/absence of a patient, and hence for switching on/off the gas pump in the respiratory gas monitor device. In the second embodiment there is a ventilator device and a breathing tube, and in the third embodiment there neither exists a ventilator device nor a breathing tube, but the gas sampling is made otherwise from the patient. The alternatives are visualized by dashed line of the ventilator device/breathing tube.

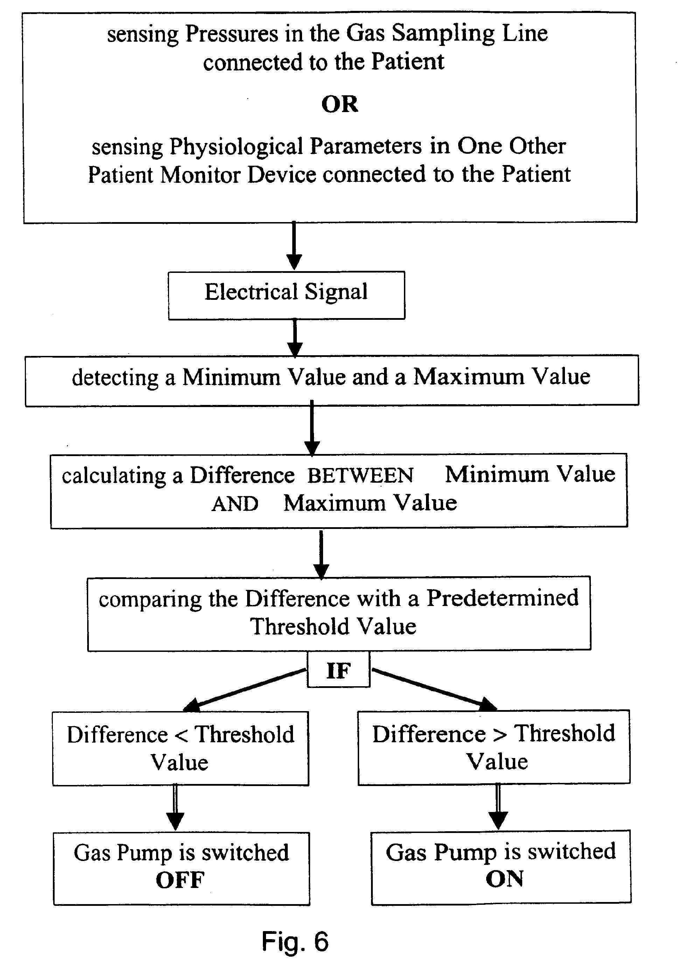

Fig. 6 shows the main steps of the method according to the invention.

### DETAILED DESCRIPTION OF THE PREFERRED EMBODIMENTS

The known respiratory gas monitor (= RGM) devices 1 comprise at least one respiratory gas analyzer 16 of a type applicable to analyze a gas component or gas components of the respiratory gas, i.e. gas components like CO₂ and/or oxygen and/or NO and/or NO₂ and/or N₂O and/or other anaesthetic gas component(s), which respiratory gas is fed to the patient and/or exhaled by the patient along a breathing tube 7. For this breathing purpose one end of the breathing tube 7 is connected to a ventilator device 8 and to the other end of the breathing tube 7 is connected to the patient 9. A gas sampling line 3 that is a thinner tube is flow-connected to the wider breathing tube 7. This gas sampling line 3 can be connected to the patient 9 also without the breathing tube 7 and the ventilator device 8. Accordingly, it is question about a diverting type or side flow monitoring of a patient. Because the respiratory gas analyzer 16 and the ventilator device 8 can be of any type appropriate for the purpose and known as such, they are not explained more in detail here. The respiratory gas monitor devices 1 further comprise a gas pump 2 for sucking sample of the respiratory gas from the breathing tube 7 through the gas sampling line 3, as well as a power supple 6 for providing electrical voltage/current to the gas pump 2, and a processing unit 5 for controlling this voltage/current to the gas pump, whereupon the gas pump creates a proper sample flow of the respiratory gas from the breathing tube to the respiratory gas analyzer 16. The respiratory gas monitor device 1 also comprises a pressure sensor 4, which is pressure-connected to the gas sampling line 3 and electrical-signal-connected to the processing unit 5. This electrical signal is normally used for correcting the measuring results of respiratory gas analyzer 16. As known, all pressure sensors 4 independent of their type have parts responsive to pressure and/or pressure variations, on the basis of which the sensors provide an electrical signal proportional to the pressure or pressure changes. Of course the proportionality not necessarily mean linear dependence, but proportionality can also be nonlinear. Figure 3 shows typical air pressure and/or airflow variations of breathing as measured by the pressure sensor 4. Processing unit 5 is typically a data processor together with necessary memories and other components. Processing unit can be a computer, but this is not necessary, because a more specialized design is in many cases more practical. Such processing units are widely used in various technical fields, and accordingly, they are not explained more in detail here.

In most cases there is also some other patient monitor devices, and very often several other patient monitor devices, which is/are connected to the patient 9 under surgical operation or under medical control or the like, and which monitor device(s) is/are capable to detect values of at least one physiological signal from the patient 9. For this purpose the other patient monitor device(s) 10-12, 13-15 is/are detection-connected to said patient and electrical-signal-connected to the processing unit 5. One of the most usual of these other patient monitor devices is a Pulse Oximeter Device, which is the most usual of the photoplethysmographic devices, and which comprises at least a pulse oximeter sensor 15 that is connected with a pulse oximeter cable 14 and via a pulse oximeter interface 13 to the processing unit 5. As known, pulse oximeter sensors measure the blood amount in tissues of the patient - the tissue is typically a finger, but can also be an auricle, or any other part of the body - utilizing the transmittance or reflectance of an infrared radiation, whereupon a photoplethysmographic signal is received for further analysis. PhotoPlethysmoGraphy (= PPG) is known as such and is, accordingly, not explained more in detail here. Any kind of the photoplethysmographic devices can provide that kind of signal, which is useful for the invention. Figure 2 shows typical blood flow/pressure variations as measured by the pulse oximeter device 13-15. One other of the usual of these other patient monitor devices is a Non Invasive Blood Pressure (= NIBP) measuring apparatus, which comprises a NIBP cuff 11 e.g. around an arm or some other respective body part, which cuff 11 is automatically pressurized and de-pressurized via a NIBP connection tube 12 for sensing the systolic and the diastolic blood pressures in the NIBP logics 10. The cuff 11 and the logics 10 together can be considered to be a blood pressure sensor. The systolic and the diastolic blood pressures are then forwarded to the processing unit 5. NIBP-apparatus does not reveal the temporal variability of the blood pressure or flow, but gives a mean value integrated over a time period, which respect the pressurizing and de-pressurizing time, as a result. This is one reason why the figures do not show any curve concerning NIBP measuring results. Still one other of the usual of these other patient monitor devices is an ElectroCardioGraphic (= ECG) device or MagnetoCardioGraphic (= MCG) device, not shown in the figures, which comprise leads connected to the patient according to predetermined known lay-out. The ECG-device or MCG-device is also electrical-signal-connected to the processing unit 5. Figure 1 shows typical variations in the cardiac excitation signal as measured by an ECG-device or a MCG-device.

According to the preferred embodiment of the present invention, the connection of a patient 1 to a RGM monitor 1 is automatically identified from the electric signal obtained from the pressure sensor 4 that measures the working pressure in the gas sampling line 3. Because of numerous effects of gas pressure on concentration measurements in the breathing air, it is always necessary to measure the working pressure and take its value into account when calculating the gas concentrations displayed to the user. Thus, the method disclosed by this invention does not require any extra components or mechanical or pneumatic constructions to the RGM.

The working pressure of the gas sensors in the RGM varies with the barometric pressure that is related with both weather conditions and altitude of the location of the RGM. These changes are very slow and can be considered irrelevant from the point of view of this invention; they merely define the pressure baseline. When a patient is connected to the anesthesia machine or ventilator and is not breathing spontaneously, pressure in the patient circuit varies cyclically in the pace of ventilation given to the patient. During the inspiration phase, there is an over pressure of 10-100 mbar. During the expiration phase the pressure drops when gas flows out of the patient's lung. The peak-to-peak amplitude of the pressure signal caused by the ventilation of the patient is between 10 and 100 mbar and the frequency of the cyclic pressure waveform equals that of the Respiration Rate of the ventilation. The exact waveform of the cyclic pressure variation depends on the settings of the ventilator and properties of the patients' airway and lung. When the gas pump in an RGM is not operating, there is no gas flow in the sampling line and accordingly no pressure drop that could change because of changes in the flow resistance of the sampling line. When the sample pump is running, the pressure drop across the sampling line may vary, if the flow resistance of the sampling line varies, for example when water vapor from the sample gas is condensing in the sampling line.

Due to the pressure conditions described above, the working pressure of the RGM remains on the baseline level when there is no patient connected to the RGM. When a patient is connected to the RGM, the working pressure changes in the pace τ_{A} of ventilation of the patient. According to the present invention, the need for respiratory gas monitoring is identified from the variations in the working pressure: when the working pressure stays constant, there is no need for gas monitoring and whereas cyclic changes in the working pressure indicate that gas monitoring is needed and the sample pump must be turned on.

The above-mentioned automatic identification is accomplished by analyzing the pressure signal from the pressure sensor 4 in the processing unit 5. The minimum values and maximum values of the working pressure is detected from the received pressure signal within a predetermined period of time, called hereafter the "Recording Period" RP, and comparing the difference between the maximum and minimum values to a threshold value, called hereafter the "Monitoring Threshold" MT. The values are of course stored in memories/memory in the processing unit 5 for further calculation purposes. If there is no patient connected to the RGM, the working pressure is essentially constant and the difference between its maximum and minimum values within the RP will be below the MT. In this case, the gas pump may stay off or can be switched off in order to keep the RGM in the Standby mode or switch it to that mode. If the gas sampling line is connected to the airway of a patient to be monitored, the working pressure will vary at the pace τ_{A} of respiration and the difference between maximum and minimum pressures during the RP will exceed the MT. In this case, the gas pump must stay on or be switched on in order to keep the RGM in its normal operating mode or automatically switch it to that mode.

Accordingly, a minimum value *Min* and a maximum value *Max* in the signal Y forwarded by the pressure sensor 4 to the processing unit 5 are detected by the processing unit 5, which minimum value *Min* and maximum value *Max* represent the minimum pressure(s) and the maximum pressure(s) respectively inside said gas sampling line 3. When the signal Y is periodically variable because of intrinsic physiological reasons - i.e. pulsing or alternating or between these like blood flow signal from PPG - a mean minimum value *Min* and a mean maximum value *Max* is calculated over the recording period RP. In this kind of cases - signal variable because of intrinsic physiological reasons - the recording period RP is several times the pace of the pulses, typically RP > 10xτ_{A}. Accordingly, differences between single pulses are eliminated. Next a difference *Max-Min* between the minimum value and the maximum value is calculated, and is compared with a predetermined threshold value MT in said processing unit 5. If the calculated difference is below the threshold value, i.e. *Max-Min* < MT, the pump 2 is switched off or kept in the prevailing non-operating state by the processing unit 5 utilizing e.g. the power source 6. Alternatively, if the calculated difference is over the threshold value, i.e. *Max-Min* > MT, the pump 2 is switched on or kept in the prevailing operating state by the processing unit 5 utilizing e.g. the power source 6.

The need for respiratory gas monitoring can be identified separately for successive recording periods RP, which can be consecutively separate, i.e. follow each other as separate periods, or have temporal overlap, i.e. can have some common pulses, whereupon the periods can also have a moving time window as the RP. In this case, the most recent data points representing the working pressure are included to the calculations for maxima and minima of the working pressure and the oldest data points are simultaneously dropped out from these calculations. The recording periods RP for turning the gas pump off RP_{OFF} and on RP_{ON} can differ in their durations. In order not to turn the gas pump off too quickly, the RP_{OFF} can be 2 - 5 minutes. In order to turn the gas pump on soon enough after the connection of a patient to the RGM, the RP_{ON} can be 5 - 50 seconds.

In many cases, an RGM has the capability to monitor other one or more physiological signals in addition to respiratory signals. In order to monitor these other signals, sensors or electrodes generating or capturing the corresponding physiological signals are connected to such an RGM. These signals can for example originate from a Pulse Oximetry sensor for monitoring the Oxygen saturation of blood often called as SpO₂, blood pressure sensor for Blood Pressure Monitoring called as BP or electrodes for ElectroCardioGraphic Monitoring called ECG or MagnetoCardiaGraphic Monitoring called MCG. The need for respiratory monitoring can be detected from one or more of the physiological signals mentioned above. The detection can be done registering the minimum and maximum values as described next.

According to two further embodiments of the invention the connection of a patient 1 to a RGM monitor 1 is automatically identified from the electric signal Y obtained either from the pulse oximeter sensor 15 - or some other sensor working according to an analogous principle - that measures the blood flow in the tissue of the patient 9 providing e.g. a PPG-signal, or from ECG-device or MCG-device that measures the cardiac excitation from the patient 9 providing a physiological electrical/magnetic signal Y. In these cases the signals are attained by different means from the body of the patient 9, but all of them somehow describe the working of patient's heart, and accordingly these signals are called with a common name "Heart Signal". The same procedure as in measuring pressures of the breathing air, described above, is also valid for these latter cases, where heart signal is received and used, i.e. only slow changes need to be taken account, and the variations at the paces τ_{A} of heart pulses are omitted. ECG and MCG are considered to be sensors, too. The above-mentioned automatic identification is accomplished by analyzing the heart signal from the Pulse Oximeter or ECG-device or MCG-device in the processing unit 5. The minimum values and maximum values of the heart signal Y is detected within a predetermined recording period RP, and comparing the difference between the maximum and minimum values to a threshold value MT. Accordingly, a minimum value *Min* and a maximum value *Max* in the signal Y forwarded by the sensor to the processing unit 5 are detected by the processing unit 5, which minimum value *Min* and maximum value *Max* represent the minimum blood flow(s) or minimum cardiac excitation(s) and the maximum blood flow(s) or maximum cardiac excitation(s) respectively in the patient. This signal being periodically variable because of intrinsic physiological reasons - i.e. pulsing or alternating or between these like blood flow signal Y from PPG - a mean minimum value *Min* and a mean maximum value *Max* is calculated over the recording period RP. Here too, the recording period RP is several times the pace of the pulses, typically RP > 10xτ_{A}. Accordingly, differences between single pulses are eliminated. Next a difference *Max-Min* between the minimum value and the maximum value is calculated, and is compared with a predetermined threshold value MT in said processing unit 5. If the calculated difference is below the threshold value, i.e. *Max-Min* < MT, the pump 2 is switched off or kept in the prevailing non-operating state by the processing unit 5 utilizing e.g. the power source 6. Alternatively, if the calculated difference is over the threshold value, i.e. *Max-Min* > MT, the pump 2 is switched on or kept in the prevailing operating state by the processing unit 5 utilizing e.g. the power source 6.

According to still one further embodiment of the invention the connection of a patient 1 to a RGM monitor 1 is automatically identified from the blood pressure signal from non-invasive blood pressure (= NIBP) measuring apparatus 10-12, which through pressurizing and de-pressurizing of the cuff aided by detection of pulse stopping and restarting provides the systolic and the diastolic blood pressure signal. The attained systolic blood pressure value is directly the mean maximum value *Max* of the signal and the attained diastolic blood pressure value is directly the mean minimum value *Min* of the signal, which are either already in or forwarded to the processing unit 5. Here the pressurizing and de-pressurizing times together form the predetermined recording period RP. From this point onwards the rest of the steps are same as performed in the other embodiments. Accordingly, a difference *Max-Min* between the minimum value and the maximum value is calculated, and is compared with a predetermined threshold value MT in said processing unit 5. If the calculated difference is below the threshold value, i.e. *Max-Min* < MT, the pump 2 is switched off or kept in the prevailing non-operating state by the processing unit 5 utilizing e.g. the power source 6. Alternatively, if the calculated difference is over the threshold value, i.e. *Max-Min* > MT, the pump 2 is switched on or kept in the prevailing operating state by the processing unit 5 utilizing e.g. the power source 6.

To avoid errors caused by self-generated noise and by electrical and magnetic fields in the environment and/or by possible other disturbances from outside the respiratory gas monitor device and/or the mentioned at least one other patient monitor device capable to detect values of at least one physiological signal from said patient, which erroneous/disturbing "signal" may be fed into the RGM 1 through pressure sensor 4, through pulse oximeter sensor 15, through ECG-device or MCG-device and/or through NIBP measuring apparatus 10-12 especially when the devices are not connected to the patient, i.e. during instances when there is no patient and the pump should be switched off, the actual signal - in fact everything that could be an input from the above mentioned sensors to the processing unit 5 - is low-pass filtered before the detection of the minimum value *Min* and the maximum value *Max.* This kind of simple filtering is in most cases very effective, because noise and other disturbances have normally high frequency compared to the frequencies of the genuine signal from the sensors 4, 15, 10-11, ECG, MCG. Heart rate is in most cases at maximum about 200 pulses/minute, and breathing rate is in most cases at maximum about 100 respirations/minute, while disturbances normally have a frequency of at least 50 Hz or 60 Hz, and mainly in the order of radio frequencies. Accordingly, low-pass filtering with a cut-off frequency at maximum 10 Hz, or in the order of 5 Hz is practical and efficient.

The detection of the presence of a patient connected to the RGM can also rely on the parameter specific algorithms that derive numeric values or waveforms for a physiological parameter from the corresponding physiological signals: A patient is considered to be connected to the RGM, if a value or values or waveform for at least one physiological signal is shown on the monitor's display or is in a state enabling a detection or visualization of the value(s) or waveform, whereupon possible noise signal components or error signal components are filtered away from the raw signal available from the signal detector(s) resulting to a clean duty or utility signal. This means that if a SpO₂-monitor, a BP-monitor, an ECG-monitor or a MCG monitor gives a data or can give a data the mentioned minimum values and maximum values can be derived from this data. Normally all monitors detecting and analyzing physiological signal(s) from a patient are provided with means that include algorithm(s) to remove erroneous signal components, i.e. there is ECG-algorithm, SpO₂-algorithm etc. to filter noise and respective signal components away from the raw signal. Some monitors have so called *LEADS* OFF-alarm, in which case: **1**} IF "*LEADS OFF*"-alarm is activated or ON no electrical-signal is forwarded to the processing unit, whereupon no difference between a non-existing minimum value and a non-existing maximum value is available → meaning non-presence of a patient, or **2**} IF *"LEADS OFF"-*alarm is not activated or OFF an electrical-signal - a duty or utility signal - is forwarded to the processing unit, whereupon a difference between an existing minimum value and an existing maximum value is available → meaning presence of a patient.

Even if the RGM would only be capable of monitoring respiratory signals, it may be a part of a monitoring system that is used for monitoring both respiratory and other physiological signals. In this case, the presence of a patient connected to the monitoring system can be communicated to the RGM by at least one of the other monitors belonging to the monitoring system whenever it detects the patient or the user commands it to start the patient monitoring.

In all these cases, the potential need of the gas monitoring is identified from the behavior of the signals from sensors that are used for patient monitoring. Many RGMs or combinations of a RGM with other patient monitoring devices contain a number sensors and devices, which measure various physiological signals. The presence of a patient to be monitored can be identified from almost any of the parameter signals obtained this way, or combinations of the parameter signals.

For the purpose of the invention oxygen saturation of blood, blood pressure, blood flow, electrical heart signal, magnetic heart signal, electrical muscular signal and electrical brain signal etc. can be used. It shall be noted that the respiratory gas analyzer and the other patient monitor device can be in one unit or separate units.

## Claims

1. A method for controlling a respiratory gas monitor device (1), which comprises:
- at least one respiratory gas analyzer (16) capable to analyze a gas component or gas components of a respiratory gas,
- a gas sampling line (3) flow-connected to a breathing tube (7) suitable for feeding of said respiratory gas to and from a patient (9),
- a gas pump (2) for sucking sample(s) of respiratory gas from said breathing tube (7) through said gas sampling line (3) to said respiratory gas analyzer (16),
- a power supply (6) and a processing unit (5), and
- a pressure sensor (4) electrical-signal-connected to said processing unit (5);
**characterized in that** said pressure sensor (4) is pressure-connected to said gas sampling line (3), and **in that**:
- a minimum value (*Min*) and a maximum value *(Max)* in the signal forwarded by said pressure sensor (4) are detected by said processing unit (5) within a predetermined recording period (RP), said recording period being several times the pace (τ_{A}) of respiration pulses, and said values (*Min* and *Max*) representing a minimum pressure and a maximum pressure respectively inside said gas sampling line (3);
- a difference (Max-Min) between said minimum value *(Min)* and said maximum value *(Max)* is calculated, and is compared with a predetermined threshold value (MT) programmed in or to said processing unit (5); and
- if said calculated difference *(Max-Min)* is:
- below said threshold value (MT) said pump (2) is switched off or kept non-operating by said processing unit (5), and
- above said threshold value (MT) said pump (2) is switched on or kept operating by said processing unit (5).

2. A method of claim 1, **characterized in that** said recording period (RP) is predetermined to be a longer recording period for turning the gas pump off, and a shorter recording period for turning the gas pump on.

3. A method of claim 2, **characterized in that** said longer recording period for turning the gas pump off is from 2 to 5 minutes.

4. A method of claim 2, **characterized in that** said shorter recording period for turning the gas pump on is from 5 to 50 seconds.

5. A method of claim 1, **characterized in that** there is a plurality of said recording periods (RP) following each other in succession.

6. A method of claim 5, **characterized in that** said successive recording periods (RP) are arranged to be consecutively separate.

7. A method of claim 1, **characterized in that** said signal forwarded by said pressure sensor (4) is low-pass filtered before said detection of the minimum value (*Min*) and the maximum value (*Max*).

8. A method for controlling a respiratory gas monitor device (1) in a patient monitoring system for monitoring both respiratory and other physiological signals, which system comprises in said respiratory gas monitor device (1):
- at least one respiratory gas analyzer (16) capable to analyze a gas component or gas components of a respiratory gas,
- a gas sampling line (3) flow-connected to a patient (9),
- a gas pump (2) for sucking sample(s) of said respiratory gas from the patient through said gas sampling line (3) to said respiratory gas analyzer (16), and
- a power supply (6) and a processing unit (5)];
**characterized in that** said system further comprises at least one patient monitor device (10-12, 13-15) capable to detect values of at least one physiological signal from said patient, said patient monitor detection-connected to said patient and electrical-signal-connected to said processing unit (5); and **in that** in said method:
- a minimum value (Min) and a maximum value (*Max*) of said one physiological signal are detected within a predetermined recording period (RP), said recording period being several times the pace (τ_{A}) of the physiological signal's pulses, by said patient monitor (10-12, 13-15);
- a difference (*Max*-*Min*) between said minimum value (Min) and said maximum value (*Max*) is calculated, and is compared with a predetermined threshold value programmed (MT) in/to said processing unit (5); and
- if said calculated difference is:
- below said threshold value (MT) said pump is switched off or kept non-operating by said processing unit (5), and
- above said threshold value (MT) said pump is switched on or kept operating by said processing unit (5).

9. A method of claim 8, **characterized in that** said patient monitor device (10-12, 13-15) detects a physiological signal selected from a group of signals including at least oxygen saturation of blood, blood pressure, blood flow, electrical heart signal, magnetic heart signal, electrical muscular signal and electrical brain signal.

10. A method of claim 8, **characterized in that** said recording period is predetermined to be a longer recording period for turning the gas pump off, and a shorter recording period for turning the gas pump on.

11. A method of claim 10, **characterized in that** said longer recording period for turning the gas pump off is from 2 to 5 minutes.

12. A method of claim 10, **characterized in that** said shorter recording period for turning the gas pump on is from 5 to 50 seconds.

13. A method of claim 8, **characterized in that** there is a plurality of said recording periods (RP) following each other in succession.

14. A method of claim 13, **characterized in that** said successive recording periods (RP) are arranged to be consecutively separate.

15. A method of claim 8, **characterized in that** said signal forwarded by said one patient monitor device (10-12, 13,15) is low-pass filtered before said detection of the minimum value (*Min*) and the maximum value *(Max).*

16. A method of claim 8, **characterized in that** said patient is free breathing or breathing via a breathing tube (7),

17. A method of claim 8, **characterized in that** said respiratory gas analyzer (16) and said at least one patient monitor device (10-12, 13-15) are a single unit or separate units.

## Patentansprüche

1. Verfahren zur Steuerung einer Atemgas-Überwachungsvorrichtung (1), aufweisend:
- mindestens einen Atemgas-Analysator (16) zur Analyse eines Gasbestandteils oder von Gasbestandteilen eines Atemgases,
- eine Gas-Probenleitung (3), die strömungsleitend an einem Atemrohr (7) angeschlossen ist, das der Zufuhr des besagten Atemgases an einen Patienten (9) sowie einer Abfuhr weg von diesem dient,
- eine Gaspumpe (2) zum Ansaugen einer Probe oder Proben von Atemgas von dem Atemrohr (7) durch die besagte Gas-Probenleitung (3) an den Atemgas-Analysator (16),
- eine Stromversorgung (6) und eine Prozessor-Einheit (5), und
- einen Drucksensor (4), der mittels elektrischer Signalleitung an die besagte Prozessor-Einheit (5) angeschlossen ist;
**dadurch gekennzeichnet, dass** der Drucksensor (4) funktional an der Gas-Probenleitung (3) angeschlossen ist, und dass:
- ein Minimalwert (Min) und ein Maximalwert (Max) in dem durch den Drucksensor (4) abgegebenen Signal durch die Prozessoreinheit (5) innerhalb einer vorbestimmten Aufnahmedauer (RP) detektiert werden, wobei diese Aufnahmedauer ein mehrfaches der Zyklenzeit (τ_{A}) von Atemstößen ist, und diese Werte (Min und Max) jeweils einen Minimaldruck und einen Maximaldruck innerhalb der Gas-Probenleitung (3) darstellen,
- eine Differenz (Max-Min) zwischen dem Minimalwert (Min) und dem Maximalwert (Max) berechnet wird, die dann mit einem vorbestimmten Schwellenwert (MT) verglichen wird, der in der Prozessor-Einheit (5) oder durch sie programmiert ist; und dass
- falls die berechnete Differenz (Max-Min):
- unterhalb des besagten Schellenwerts (MT) ist, so die Pumpe (2) ausgeschaltet oder im Ruhezustand mittels der Prozessoreinheit (5) gehalten wird, und falls sie
- oberhalb des Schwellenwertes (MT) ist, die Pumpe (2) eingeschaltet oder durch die Prozessor-Einheit (5) im Betriebsmodus gehalten wird.

2. Verfahren nach Anspruch 1, **dadurch gekennzeichnet, dass** die Aufnahmedauer (RP) zum Ausschalten der Gaspumpe auf eine längere Aufnahmedauer vorbestimmt ist, und zum Einschalten der Gaspumpe auf eine kürzere Aufnahmedauer.

3. Verfahren nach Anspruch 2, **dadurch gekennzeichnet, dass** die längere Aufnahmedauer zum Ausschalten der Gaspumpe von 2 bis 5 Minuten beträgt.

4. Verfahren nach Anspruch 2, **dadurch gekennzeichnet, dass** die kürzere Aufnahmedauer zum Einschalten der Gaspumpe von 5 - 50 Sekunden dauert.

5. Verfahren nach Anspruch 1, **dadurch gekennzeichnet, dass** es eine Mehrzahl von Aufnahmedauern (RP) gibt, die sich jeweils in einer Abfolge folgen.

6. Verfahren nach Anspruch 5, **dadurch gekennzeichnet, dass** die aufeinanderfolgenden Aufnahmedauern (RP) so bestimmt sind, dass sie konsekutiv separat vorliegen.

7. Verfahren nach Anspruch 1, **dadurch gekennzeichnet, dass** das an den Drucksensor (4) weitergegebene Signal Tiefpass-gefiltert ist, bevor die besagte Detektion des Minimalwerts (Min) und des Maximalwerts (Max) erfolgt.

8. Verfahren zur Steuerung einer Atemgas-Überwachungsvorrichtung (1) in einem Patienten-Überwachungssystem zur Überwachung von sowohl der Atmung als auch anderen physiologischen Signalen, welches System in der Atemgas-Überwachungsvorrichtung (1) aufweist:
- mindestens einen Atemgas-Analysator (16), der zur Analyse eines Gasbestandteils oder von Gasbestandteilen eines Atemgases dient,
- eine Gas-Probenleitung (3), die strömungsleitend an einem Patienten (9) angeschlossen ist,
- eine Gaspumpe (2) zum Ansaugen einer Probe oder mehreren Proben des Atemgases von dem Patienten durch die besagte Gas-Probenleitung (3) an den besagten Atemgas-Analysator (16), und
- eine Stromversorgung (16) und eine Prozessor-Einheit (5);
**dadurch gekennzeichnet, dass** das System ferner mindestens eine Patientenüberwachungsvorrichtung (10-12, 13-15) aufweist, die der Detektierung von Werten von mindestens einem physiologischen Signal des besagten Patienten dient, wobei die Patientenüberwachung funktional zur Detektierung an dem Patienten und funktional zur elektrischen Signalübertragung an der Prozessoreinheit (5) angeschlossen ist; und dass in dem Verfahren:
- ein Minimalwert (Min) und ein Maximalwert (Max) des besagten einen physiologischen Signals innerhalb einer vorbestimmten Aufnahmedauer (RP) durch den besagten Patientenmonitor (10-12, 13-15) detektiert werden, wobei diese Aufnahmedauer ein mehrfaches der Zykluszeit (τ_{A}) des physiologischen Signal-Pulses ist;
- eine Differenz (Max-Min) zwischen dem Minimalwert (Min) und dem Maximalwert (Max) berechnet wird, und diese mit einem vorprogrammierten Schwellenwert (MT) in der Prozessoreinheit (5) oder durch sie verglichen wird; und
- falls diese berechnete Differenz:
- unterhalb des besagten Schwellenwerts (MT) ist, die Pumpe ausgeschaltet wird oder durch die Prozessoreinheit (5) im Ruhezustand belassen bleibt, und
- oberhalb des besagten Schwellenwerts (MT) ist, die Pumpe eingeschaltet wird oder durch die besagte Prozessoreinheit (5) im Betriebszustand belassen bleibt.

9. Verfahren nach Anspruch 8, **dadurch gekennzeichnet, dass** die Patientenüberwachungsvorrichtung (10-12, 13-15) ein physiologisches Signal detektiert, das ausgewählt ist aus einer Gruppe von Signalen, einschließlich von zumindest der Sauerstoffsättigung des Blutes, des Blutdrucks, der Blutströmung, des elektrischen Herzsignals, des magnetischen Herzsignals, des elektrischen Muskelsignals und des elektrischen Gehirnsignals.

10. Verfahren nach Anspruch 8, **dadurch gekennzeichnet, dass** die Aufnahmedauer zum Ausschalten der Gaspumpe auf eine längere Aufnahmedauer vorbestimmt ist, und zum Einschalten der Pumpe auf eine kürzere Aufnahmedauer.

11. Verfahren nach Anspruch 10, **dadurch gekennzeichnet, dass** die längere Aufnahmedauer zum Ausschalten der Gaspumpe von 2 bis 5 Minuten beträgt.

12. Verfahren nach Anspruch 10, **dadurch gekennzeichnet, dass** die kürzere Aufnahmedauer zum Einschalten der Gaspumpe von 5 bis 50 Sekunden dauert.

13. Verfahren nach Anspruch 8, **dadurch gekennzeichnet, dass** eine Mehrzahl an Aufnahmedauern (RP) vorliegt, die nacheinander folgen.

14. Verfahren nach Anspruch 13, **dadurch gekennzeichnet, dass** die aufeinanderfolgenden Aufnahmedauern (RP) konsekutiv separat vorliegen.

15. Verfahren nach Anspruch 8, **dadurch gekennzeichnet, dass** das durch die Patientenüberwachungsvorrichtung (10-12, 13-15) weitergeleitete Signal Tiefpass-gefiltert wird, bevor der Minimalwert (Min) und der Maximalwert (Max) detektiert werden.

16. Verfahren nach Anspruch 8, **dadurch gekennzeichnet, dass** der Patient via eines Atemrohres (7) frei atmet atmet.

17. Verfahren nach Anspruch 8, **dadurch gekennzeichnet, dass** der Atemgas-Analysator (16) und die besagte mindestens eine Patientenüberwachungsvorrichtung (10-12, 13-15) einzelne oder voneinander getrennte Einheiten sind.

## Revendications

1. Procédé pour contrôler un moniteur de gaz respiratoire (1), qui comprend :
- au moins un analyseur de gaz respiratoire (16) capable d'analyser un composant gazeux ou des composants gazeux d'un gaz respiratoire,
- une tuyauterie d'échantillonnage de gaz (3) raccordée à écoulement à un tube respiratoire (7) conçu pour le passage dudit gaz respiratoire destiné à un patient (9) ou provenant de celui-ci,
- une pompe à gaz (2) pour aspirer l'échantillon ou les échantillons de gaz respiratoire à partir dudit tube respiratoire (7) à travers ladite tuyauterie d'échantillonnage de gaz (3) vers ledit analyseur de gaz respiratoire (16),
- une source d'alimentation (6) et une unité de traitement (5), et
- un capteur de pression (4) raccordé par signal électrique à ladite unité de traitement (5) ; **caractérisé en ce que** ledit capteur de pression (4) est raccordé par pression à ladite tuyauterie d'échantillonnage de gaz (3), et **en ce que** :
- une valeur minimale (*Min*) et une valeur maximale (*Max*) dans le signal transmis par ledit capteur de pression (4) sont détectées par ladite unité de traitement (5) dans une période d'enregistrement prédéterminée (RP), ladite période d'enregistrement étant égale à plusieurs fois la cadence (τ_{A}) des impulsions respiratoires, et lesdites valeurs (*Min* et *Max*) représentant respectivement une pression minimale et une pression maximale à l'intérieur de ladite tuyauterie d'échantillonnage du gaz (3),
- une différence (*Max - Min*) entre ladite valeur minimale (*Min*) et ladite valeur maximale (*Max*) est calculée, et est comparée à une valeur de seuil prédéterminée (MT) programmée dans ou vers ladite unité de traitement (5) ; et
- si ladite différence calculée (*Max - Min*) est :
- en dessous de ladite valeur de seuil (MT) ladite pompe (2) est arrêtée ou maintenue en état de non-fonctionnement par ladite unité de traitement (5), et
- au-dessus de ladite valeur de seuil (MT), ladite pompe (2) est mise en marche ou maintenue en état de fonctionnement par ladite unité de traitement (5).

2. Procédé selon la revendication 1, **caractérisé en ce que** ladite période d'enregistrement (RP) est prédéterminée comme devant être une période d'enregistrement plus longue pour la désactivation de la pompe à gaz, et une période d'enregistrement plus courte pour l'activation de la pompe à gaz.

3. Procédé selon la revendication 2, **caractérisé en ce que** ladite période d'enregistrement plus longue pour l'activation de la pompe à gaz va de 2 à 5 minutes.

4. Procédé selon la revendication 2, **caractérisé en ce que** ladite période d'enregistrement plus courte pour l'activation de la pompe à gaz va de 5 à 50 secondes.

5. Procédé selon la revendication 1, **caractérisé en ce qu'**il existe une pluralité desdites périodes d'enregistrement (RP) se suivant successivement.

6. Procédé selon la revendication 5, **caractérisé en ce que** lesdites périodes d'enregistrement (RP) successives sont agencées de sorte à être consécutivement distinctes.

7. Procédé selon la revendication 1, **caractérisé en ce que** ledit signal transmis par ledit capteur de pression (4) fait l'objet d'une filtration passe-bas avant ladite détection de la valeur minimale (Min) et de la valeur maximale (*Max*).

8. Procédé de contrôle d'un moniteur de gaz respiratoire (1) dans un système de surveillance de patient destiné à surveiller à la fois les signaux respiratoires et d'autres signaux physiologiques, lequel système comprend dans ledit moniteur de gaz respiratoire (1) :
- au moins un analyseur de gaz respiratoire (16) capable d'analyser un composant gazeux ou des composants gazeux d'un gaz respiratoire,
- une tuyauterie d'échantillonnage de gaz (3) raccordée par débit à un patient (9),
- une pompe à gaz (2) pour aspirer l'échantillon ou les échantillons dudit gaz respiratoire provenant du patient à travers ladite tuyauterie d'échantillonnage de gaz (3) vers ledit analyseur de gaz respiratoires (16), et
- une source d'alimentation (6) et une unité de traitement (5) ;
**caractérisé en ce que** ledit système comprend en outre au moins un moniteur de patient (10-12, 13-15) capable de détecter des valeurs d'au moins un signal physiologique dudit patient, ledit moniteur de patient étant raccordé par détection audit patient et raccordé par signal électrique à ladite unité de traitement (5) ; et **en ce que** dans ledit procédé :
- une valeur minimale (Min) et une valeur maximale (*Max*) dudit un signal physiologique sont détectées dans une période d'enregistrement (RP) prédéterminée, ladite période d'enregistrement étant égale à plusieurs fois la cadence (τ_{A}) des impulsions du signal physiologique, par ledit moniteur de patient (10-12, 13-15) ;
- une différence (*Max - Min*) entre ladite valeur minimale (Min) et ladite valeur maximale (*Max*) est calculée, et est comparée à une valeur de seuil (MT) prédéterminée programmée dans/vers ladite unité de traitement (5) ; et
- si ladite différence calculée est :
- en dessous de ladite valeur de seuil (MT), ladite pompe est arrêtée ou maintenue en état de non-fonctionnement par ladite unité de traitement (5); et
- au-dessus de ladite valeur de seuil (MT), ladite pompe est mise en marche ou maintenue en état de fonctionnement par ladite unité de traitement (5).

9. Procédé selon la revendication 8, **caractérisé en ce que** ledit moniteur de patient (10-12, 13-15) détecte un signal physiologique choisi dans un groupe de signaux comprenant au moins la saturation en oxygène du sang, la tension artérielle, le débit sanguin, le signal électrique cardiaque, le signal magnétique cardiaque, le signal électrique musculaire et le signal électrique cérébral.

10. Procédé selon la revendication 8, **caractérisé en ce que** ladite période d'enregistrement est prédéterminée comme devant être une période d'enregistrement plus longue pour la désactivation de la pompe à gaz, et une période d'enregistrement plus courte pour l'activation de la pompe à gaz.

11. Procédé selon la revendication 10, **caractérisé en ce que** ladite période d'enregistrement plus longue pour la désactivation de la pompe à gaz va de 2 à 5 minutes.

12. Procédé selon la revendication 10, **caractérisé en ce que** ladite période d'enregistrement plus courte pour l'activation de la pompe à gaz va de 5 à 50 secondes.

13. Procédé selon la revendication 8, **caractérisé en ce qu'**il existe une pluralité desdites périodes d'enregistrement (RP) se suivant successivement.

14. Procédé selon la revendication 13, **caractérisé en ce que** lesdites périodes d'enregistrement (RP) successives sont agencées de sorte à être consécutivement distinctes.

15. Procédé selon la revendication 8, **caractérisé en ce que** ledit signal envoyé par ledit un moniteur de patient (10-12, 13-15) fait l'objet d'une filtration passe-bas avant ladite détection de la valeur minimale (*Min*) et de la valeur maximale (Max).

16. Procédé selon la revendication 8, **caractérisé en ce que** ledit patient respire librement ou respire par le biais d'un tube respiratoire (7).

17. Procédé selon la revendication 8, **caractérisé en ce que** ledit analyseur de gaz respiratoires (16) et ledit au moins un moniteur de patient (10-12, 13-15) sont une seule unité ou des unités distinctes.
